# EUROPEAN PATENT SPECIFICATION

(11) **EP 4 351 367 B1**
(45) Date of publication and mention of the grant of the patent: **20.08.2025**
(21) Application number: 23793215.7
(22) Date of filing: 18.08.2023
(51) Int. Cl.: A24F 40/05, A24F 40/50, A61M 15/00, A24F 40/20, A24F 40/51, A24F 40/485, A61M 11/00, A61M 11/02, A61M 16/00

(54) **POWDER INHALATION DEVICE AND CONTROL METHOD THEREOF**
PULVERINHALATIONSVORRICHTUNG UND STEUERUNGSVERFAHREN DAFÜR
DISPOSITIF D'INHALATION DE POUDRE ET SON PROCÉDÉ DE COMMANDE

(30) Priority: 23.08.2022 KR 20220105638
(43) Date of publication of application: 17.04.2024
(73) Proprietor: KT&G Corporation, Daedeok-gu Daejeon 34337 (KR)
(72) Inventor: KIM, Min Kyu, Seoul 08211 (KR); LEE, Won Kyeong, Guri-si Gyeonggi-do 11920 (KR)
(74) Representative: AWA Sweden AB
(86) International application number: PCT/KR2023/012298
(87) International publication number: WO 2024/043630

(56) References cited:
- WO-A1-2021/243944
- WO-A1-2022/112781
- KR-A- 20190 051 785
- KR-A- 20210 101 980
- US-A- 5 469 843
- US-A1- 2003 164 169
- US-A1- 2017 119 981
- US-A1- 2019 387 794

## Description

### [Technical Field]

Embodiments of the disclosure relate to a powder inhalation device through which powder is aerosolized to allow a user to inhale the powder, and a method of controlling the powder inhalation device.

### [Background Art]

A powder inhalation device is a device for aerosolizing powder and supplying the aerosolized powder to a user such as administering a powder-type drug for treating asthma and other respiratory diseases to a user or providing powder containing nicotine to a user.

A sufficient amount of aerosolized powder should be supplied according to the purpose.

Conventional powder inhalation devices are passively operated according to users' inhalation efforts to inhale powder. However, inhalation abilities may vary according to users, and thus the powder may not be sufficiently supplied when powder is inhaled by passively operating powder inhalation devices.

In particular, when a user's inhalation ability is insufficient, powder may not be sufficiently supplied to the user.

Also, when powder agglomerates or becomes sticky due to the nature of the powder, the flow of air may be poor and the powder may not be sufficiently supplied.

US2003/164169 A1 (STANGL) relates to an inhalation device comprising an oscillator, on which a powder for nebulisation may be placed, containing a medicament and which may be activated in order to nebulise the applied powder, a mixing chamber into which the powder particles are thrown and where an aerosol cloud is formed from incoherent particles, an inhalation check valve which permits ambient air into the mixing chamber during inhalation to evacuate the particle cloud and an exhalation check valve, by means of which expired air is led off into the environment to prevent damp breath entering the mixing chamber during exhalation.

US 2017/119981 A1 (DAVIDSON PERRY) relates to an inhaler device for pulmonary delivery of at least one substance from a drug dose cartridge to an inhaling user, comprising: a first conduit for conducting a carrier airflow to a proximal opening of a mouthpiece for use by the user; a holder configured to position the dose cartridge within the carrier airflow; and a second conduit for conducting a shunting airflow to the mouthpiece without passing through the dose cartridge position. It can also be said to relate to a controller connected to a valve that controls a rate of carrier airflow, for example by controlling the shunting airflow, based on a sensor indication of airflow rate and a target airflow profile.

Accordingly, there is a need for a powder inhalation device through which a sufficient amount of powder may be reliably supplied to a user according to the user's inhalation ability.

### [Disclosure]

### [Technical Problem]

An objective of a powder inhalation device according to various embodiments of the disclosure is to stably supply powder in accordance with a user's inhalation ability.

Also, an objective of a powder inhalation device according to various embodiments of the disclosure is to supply sufficient powder to a user even when the user's inhalation ability is insufficient.

Also, an objective of a powder inhalation device according to various embodiments of the disclosure is to supply powder to a user in accordance with the user's inhalation timing.

Technical objectives to be achieved by embodiments of the disclosure are not limited thereto, and other unmentioned technical objectives will be apparent to one of ordinary skill in the art from the present specification and the accompanying drawings.

### [Technical Solution]

A powder inhalation device according to claim 1.

A method of controlling a powder inhalation device according to claim 9.

### [Advantageous Effects]

A powder inhalation device according to various embodiments of the disclosure may stably supply powder to a user in accordance with the user's inhalation ability.

Also, a powder inhalation device according to various embodiments of the disclosure may supply sufficient powder to a user even when the user's inhalation ability is insufficient.

Also, a powder inhalation device according to various embodiments of the disclosure may supply powder to a user in accordance with the user's inhalation timing.

The effects according to one or embodiments are not limited to the effects described above, and unmentioned effects will be clearly understood by one of ordinary skill in the art from the present specification and the accompanying drawings.

### [Description of Drawings]

FIG. 1 is a schematic view illustrating a powder inhalation device, according to an embodiment.
FIG. 2 is a schematic view illustrating a powder inhalation device, according to another embodiment.
FIG. 3 is a schematic view illustrating a powder inhalation device, according to another embodiment.
FIG. 4 is a schematic view illustrating a powder inhalation device, according to another embodiment.
FIG. 5 is a schematic view illustrating a powder inhalation device, according to another embodiment.
FIG. 6 is a block diagram illustrating a powder inhalation device, according to an embodiment.
FIG. 7 is a graph for describing an operation of a powder inhalation device, according to an embodiment.
FIG. 8 is a graph for describing an operation of a powder inhalation device, according to another embodiment.
FIG. 9 is a flowchart for describing a method of controlling a powder inhalation device, according to an embodiment.
FIG. 10 is a flowchart for describing a method of controlling a powder inhalation device, according to another embodiment.
FIG. 11 is a graph for describing a method by which a powder inhalation device supplies sufficient powder in accordance with a user's inhalation ability, according to an embodiment.

### [Best Mode]

Regarding the terms in the various embodiments, the general terms which are currently and widely used are selected in consideration of functions of structural elements in the various embodiments of the present disclosure. However, meanings of the terms can be changed according to intention, a judicial precedence, the appearance of a new technology, and the like. In addition, in certain cases, terms which can be arbitrarily selected by the applicant in particular cases. In such a case, the meaning of the terms will be described in detail at the corresponding portion in the description of the present disclosure. Therefore, the terms used in the various embodiments of the present disclosure should be defined based on the meanings of the terms and the descriptions provided herein.

In addition, unless explicitly described to the contrary, the word "comprise" and variations such as "comprises" or "comprising" will be understood to imply the inclusion of stated elements but not the exclusion of any other elements. In addition, the terms "-er", "-or", and "module" described in the specification mean units for processing at least one function and operation and can be implemented by hardware components or software components and combinations thereof.

As used herein, when an expression such as "at least any one" precedes arranged elements, it modifies all elements rather than each arranged element. For example, the expression "at least any one of a, b, and c" should be construed to include a, b, c, or a and b, a and c, b and c, or a, b, and c.

When two variables are proportional to each other, it should be interpreted that the two variables have any relation in which when one variable increases, the other variable increases, and when one variable decreases, the other variable decreases, including not only a linear functional relation but also any other proportional relations such as a quadratic functional relation.

An embodiment of the disclosure will now be described more fully with reference to the accompanying drawings for one of ordinary skill in the art to be able to perform the embodiment of the disclosure without any difficulty.

The disclosure is described with reference to various embodiments of powder inhalation devices, but may be embodied in many different forms and should not be construed as limited to the embodiments described herein.

Hereinafter, embodiments of the disclosure will be described in detail with reference to the drawings.

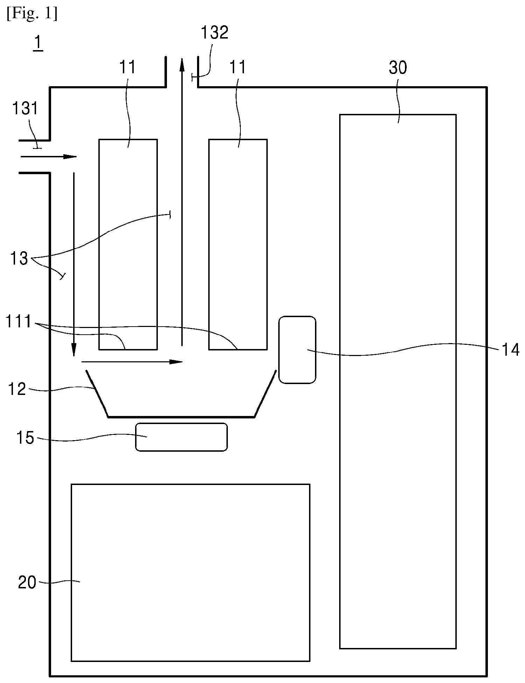

FIGS. 1 to 5 are schematic views illustrating a powder inhalation device, according to various embodiments of the disclosure.

An internal structure of a powder inhalation device 1 is not limited to that shown in FIGS. 1 to 5. That is, according to a design of the powder inhalation device 1, some of elements illustrated in FIGS. 1 to 5 may be omitted or new elements may be further added.

FIG. 1 is a schematic view illustrating a powder inhalation device, according to an embodiment.

Referring to FIG. 1, the powder inhalation device 1 may include a storage tank 11, a powder loading unit 12, an air flow passage 13, an air flow sensor 14, a powder diffuser 15, a controller 20, and a battery 30.

The air flow passage 13 may include an air flow inlet 131 and an air flow outlet 132.

When a user using the powder inhalation device 1 performs an operation of inhaling air by using the air flow outlet 132, external air is inhaled into the air flow passage 13 through the air flow inlet 131. The air inhaled into the air flow passage 13 may be discharged to the user through the air flow outlet 132.

Because an air flow through the air flow passage 13 flows from the air flow inlet 131 toward the air flow outlet 132, the air flow flowing through the air flow passage 13 may be referred to as upstream when it is close to the air flow inlet 131 and may be referred to as downstream when it is close to the air flow outlet 132.

For better understanding, directions are defined as follows.

A direction in which an air flow is discharged to the air flow outlet 132 of the powder inhalation device 1 may be referred to as an upward direction, and the opposite direction may be referred to as a downward direction. The directions defined herein may be used not only for the description of the embodiment of FIG. 1 but also for the description of the entire disclosure.

A structure of the air flow passage 13 and an arrangement of the air flow inlet 131 and the air flow outlet 132 shown in FIG. 1 are exemplary, and various structures in which an air flow is introduced into the powder inhalation device 1 and is discharged to the outside may be applied to the disclosure.

The powder inhalation device 1 may include a storage tank 11. The storage tank 11 is an element having an inner space therein, and a shape of the storage tank 11 is not limited. A powder-type medium may be stored in the storage tank 11. The powder-type medium stored in the storage tank 11 may move in the storage tank 11.

The powder-type medium may include nicotine.

In another example, the powder-type medium may include a drug for treating respiratory disease for asthma patients.

A storage tank outlet 111 connected to the inner space of the storage tank 11 may be formed in the storage tank 11. The powder-type medium accommodated in the storage tank 11 may be discharged to the outside of the storage tank 11 through the storage tank outlet 111.

According to an embodiment, the storage tank outlet 111 may be formed at the bottom of the storage tank 11. In this arrangement, the powder-type medium accommodated in the storage tank 11 may move to the bottom of the storage tank by gravity and may be discharged to the storage tank outlet 111.

A mesh or net-type structure (not shown) may be located at the storage tank outlet 111. The mesh or net-type structure (not shown) may prevent excessive discharge of the powder-type medium in the storage tank 11 and may assist in appropriate and sufficient discharge of the powder-type medium.

The powder loading unit 12 is an element for receiving the powder-type medium discharged from the storage tank 11. The powder loading unit 12 is an element for receiving and containing the powder-type medium discharged from the storage tank 11 before it is discharged to the air flow outlet 132.

According to an embodiment, the powder loading unit 12 may be located below the storage tank 11.

The powder loading unit 12 may be formed in any of various shapes capable of receiving the powder-type medium.

For example, the powder loading unit 12 may be formed in a bowl shape that is convex downward and concave upward in order to efficiently receive the powder-type medium.

In another example, the powder loading unit 12 may be formed in a flat shape.

The powder-type medium transferred to the powder loading unit 12 may be guided toward the air flow outlet 132 by an air flow flowing through the air flow passage 13.

According to an embodiment, an air flow sensor 14 may be located adjacent to the air flow passage 13. The air flow sensor 14 detects a pressure, a flow rate, and/or a flow velocity in the air flow passage 13. The air flow sensor 14 may include a pressure sensor (not shown), a flow rate sensor (not shown), and/or a flow velocity sensor (not shown).

The powder diffuser 15 is an element for diffusing the powder-type medium transferred to the powder loading unit 12 toward the air flow passage 13.

Preferably, the powder diffuser 15 may diffuser the powder-type medium transferred to the powder loading unit 12 in a downstream direction of the air flow flowing through the air flow passage 13, that is, in a direction toward the air flow outlet 132.

Referring to FIG. 1, the powder diffuser may be located below the powder loading unit 12.

The powder diffuser 15 may physically contact the powder loading unit 12.

A position of the powder diffuser 15 described with reference to the drawings is exemplary, and a position of the powder diffuser 15 is not limited thereto. That is, the powder diffuser 15 may be located at any of various positions to diffuse the powder-type medium toward the air flow passage, and such an arrangement is also included in the scope of the disclosure.

Hereinafter, the same elements as those in the powder inhalation device 1 described with reference to FIG. 1 will not be repeatedly described in detail.

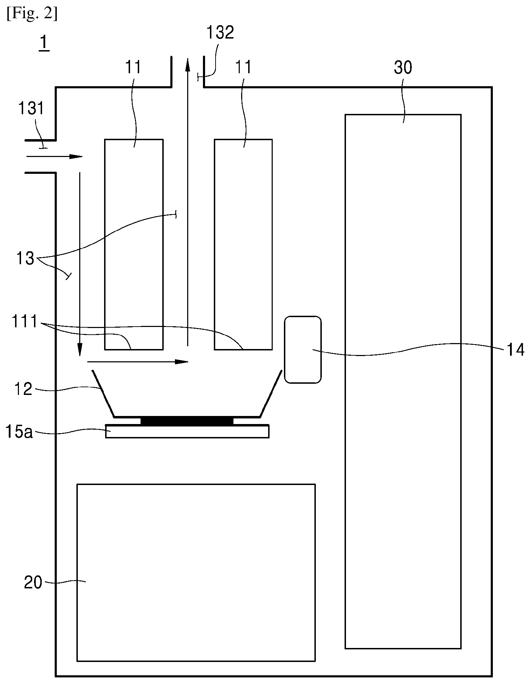

FIG. 2 is a schematic view illustrating the powder inhalation device 1, according to another embodiment.

According to the present embodiment, the powder diffuser may include a vibration device 15a for applying vibration to the powder loading unit 12.

The vibration applied by the vibration device 15a to the powder loading unit 12 refers to a repetitive operation of vibrating the powder loading unit 12 by shaking or tapping the powder loading unit 12 at pre-determined intervals.

Because the vibration device 15a vibrates the powder-type medium by vibrating the powder loading unit 12, the diffusion efficiency of the powder-type medium toward the air flow passage 13 may be maximized.

Preferably, the vibration device 15a may diffuse the powder-type medium in a downstream direction of the air flow flowing through the air flow passage 13, that is, in a direction toward the air flow outlet 132, by vibrating the powder loading unit 12.

According to the present embodiment, the powder loading unit 12 may include a vibration plate (not shown). The vibration plate may be formed in a shape and of a material capable of being vibrated by vibration generated by the vibration device 15a, transmitting the vibration generated by the vibration device 15a to the powder-type medium, and/or amplifying the vibration generated by the vibration device 15a.

For example, the vibration plate may be formed in a thin-film structure.

The vibration device 15a according to the present embodiment may be implemented in any of various methods and structures capable of vibrating the powder diffuser 15.

For example, the vibration device 15a may include a piezoelectric element (not shown) capable of converting electrical energy into pressure.

In another embodiment, the vibration device 15a may vibrate the powder diffuser 15 by using an ultrasonic vibration method.

In another example, the vibration device 15a may vibrate the powder diffuser 15 by using a motor and a cam.

In another example, the vibration device 15a may vibrate the powder diffuser 15 by using a solenoid-type actuator.

A vibration frequency of the vibration device 15a may be in a wide range of frequencies such as a low frequency of about 3 Hz to about 60 Hz and/or a high frequency of about 25 kHz to about 2 MHz.

The controller 20 may control an intensity of the vibration by controlling an amplitude, a frequency, etc. of the vibration applied by the vibration device 15a, and may control the amount of diffusion of the powder-type medium.

Accordingly, the powder inhalation device 1 may adjust a degree of diffusion of the powder-type medium when necessary by adjusting the amplitude and the frequency of the vibration.

Because the powder inhalation device 1 according to the present embodiment controls the amount of diffusion of the powder-type medium through the vibration device 15a to control a concentration of the powder-type medium included in the air flow, a sufficient powder-type medium may be supplied to the user in accordance with the user's inhalation ability and/or inhalation timing. Also, reliable supply of the powder-type medium may be ensured even when the user's inhalation ability is insufficient.

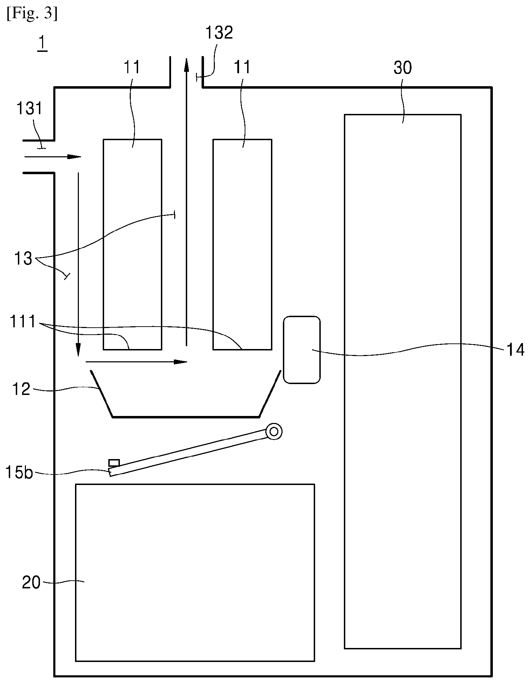

FIG. 3 is a schematic view illustrating the powder inhalation device 1, according to another embodiment.

According to the present embodiment, the powder diffuser may include an impact device 15b for applying impact to the powder loading unit 12.

The impact applied by the impact device 15b to the powder loading unit 12 may be generated by instantaneously contacting or hitting the powder loading unit 12 once or several times.

The impact applied by the impact device 15b to the powder loading unit 12 is different from vibration of the vibration device 15a of FIG. 2 in which the same operation is periodically performed a plurality of times at pre-determined intervals.

The impact device 15b may maximize the diffusion efficiency of the powder-type medium toward the air flow passage 13 by applying physical impact to the powder loading unit 12.

Preferably, the impact device 15b may diffuse the powder-type medium in a downstream direction of the air flow flowing through the air flow passage 13, that is, in a direction toward the air flow outlet 132, by applying impact to the powder loading unit 12.

As described above, the powder loading unit 12 may be concave (i.e., convex downward). In this case, a shape of the powder loading unit 12 may be temporarily deformed by impact applied by the powder diffuser 15, and the powder-type medium of the powder loading unit 12 may be more effectively diffused toward the air flow passage 13.

The impact device 15b may include, for example, a torsion spring and a switch. The torsion spring may be operated by the switch, and may be controlled to apply impact to the powder diffuser 15 by converting elastic positional energy into kinetic energy.

In another example, the impact device 15b may include an electric motor (not shown). The impact device 15b may apply impact to the powder diffuser 15 by converting rotational kinetic energy into linear kinetic energy by using an electric motor and a power transmission unit, or may apply impact to the powder diffuser 15 by using a linear electric motor that generates linear displacement.

The controller 20 may control the amount of diffusion of the powder-type medium by adjusting a frequency and strength of impact applied by the impact device 15b.

Because the powder inhalation device 1 according to the present embodiment controls the amount of diffusion of the powder-type medium through the impact device 15b to control a concentration of the powder-type medium included in the air flow, a sufficient powder-type medium may be supplied to the user in accordance with the user's inhalation ability and inhalation timing. Also, reliable supply of the powder-type medium may be ensured even when the user's inhalation ability is insufficient.

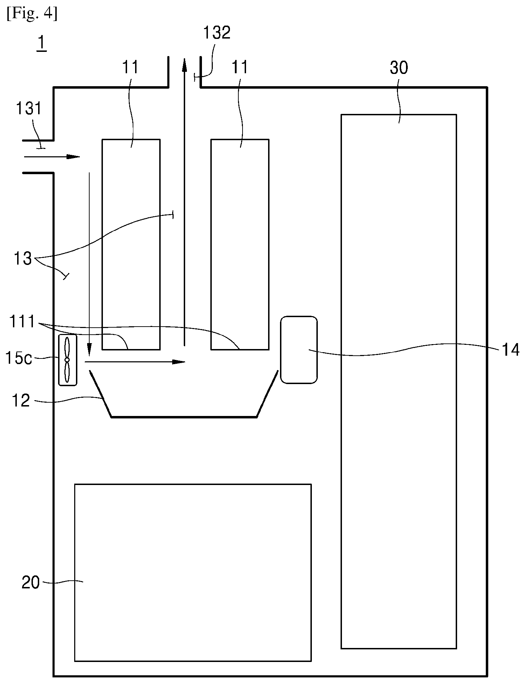

FIG. 4 is a schematic view illustrating the powder inhalation device 1, according to another embodiment.

Referring to the present embodiment, the powder diffuser may include a blower 15c for generating a flow of a fluid.

Referring to FIG. 4, the blower 15c may be located below the storage tank 11 and above the powder loading unit 12.

The blower 15c may include a rotatable blade, and may generate a flow of air by rotating the blade through power supplied from the battery 30.

When the blower 15c generates a flow of air flowing toward the powder loading unit 12, mixing of the powder-type medium of the powder loading unit 12 and air flowing through the air flow passage 13 is promoted by the flow of the air.

Preferably, the blower 15c may generate a flow of air in a downstream direction of the air flow flowing through the air flow passage 13, that is, in a direction toward the air flow outlet 132.

The amount of powder-type medium supplied to the air flow outlet 132 may be adjusted according to an intensity of the flow of the air generated by the blower 15c.

The blower 15c may be located at any of various positions to generate the flow of the air toward the powder loading unit 12. A regulator (not shown) whose opening area may be adjusted to adjust a flow rate and/or a flow velocity of the flow of the air generated by the blower 15c may be provided at an outlet of the blower 15c.

The controller 20 may control the amount of diffusion of the powder-type medium by adjusting a strength of power supplied to the blower 15c by the battery 30. In another example, the controller 20 may control the amount of diffusion of the powder-type medium by adjusting an open area of the regulator installed at the outlet of the blower 15c.

Because the powder inhalation device 1 according to the present embodiment may increase a flow rate and/or a flow velocity of the air flow passage 13 through the blower 15c to increase the amount of powder-type medium supplied to the user, a sufficient powder-type medium may be supplied to the user in accordance with the user's inhalation ability and/or inhalation timing, and reliable supply of the powder-type medium may be ensured even when the user's inhalation ability is insufficient.

Although the powder diffuser of the powder inhalation device 1 according to the embodiments described with reference to FIGS. 2, 3, and 4 includes one of the vibration device 15a, the impact device 15b, and the blower 15c, the powder diffuser may include two or more of the vibration device 15a, the impact device 15b, and the blower 15c.

For example, the powder diffuser may include the vibration device 15a and the blower 15c. In this case, the vibration device 15a may apply vibration to the powder loading unit 12 and the blower 15c may diffuse the powder-type medium to the air flow passage 13. Operations of the vibration device 15a and the blower 15c may be simultaneously performed.

In another example, the powder diffuser may include the vibration device 15a and the impact device 15b. In this case, the vibration device 15a may generate periodic vibration to the powder loading unit 12 and the impact device 15b may diffuse the powder-type medium to the air flow passage 13. Operations of the vibration device 15a and the impact device 15b may be simultaneously performed.

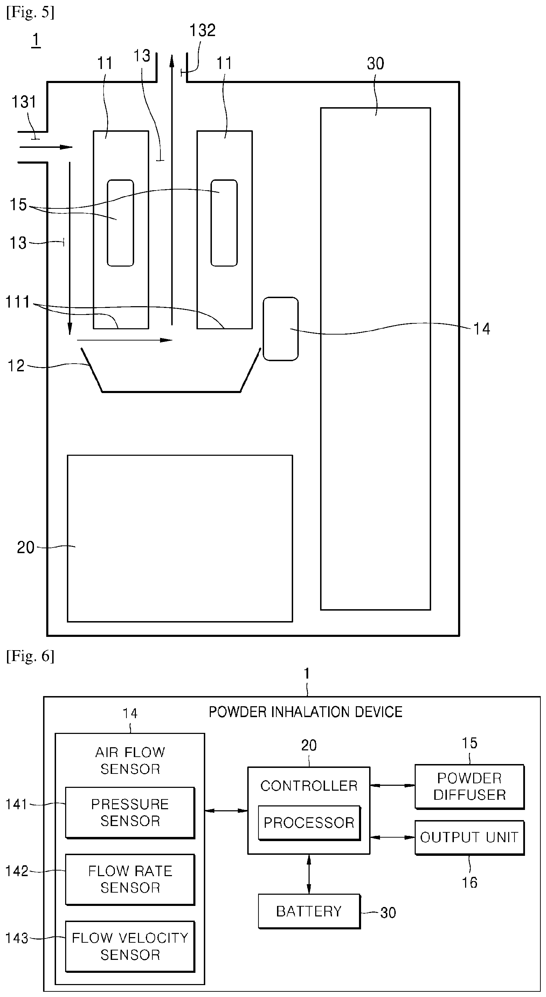

FIG. 5 is a schematic view illustrating the powder inhalation device 1, according to another embodiment.

Referring to FIG. 5, the powder diffuser 15 may be located adjacent to the storage tank 11. The powder diffuser 15 may be physically connected to the storage tank 11.

In the present embodiment, the powder diffuser 15 may include a vibration device. According to the present embodiment, the vibration device may vibrate the powder-type medium accommodated in the storage tank 11 by vibrating the storage tank 11, and thus the amount of powder-type medium transferred to the powder loading unit 12 may be increased.

An operation of the vibration device according to the present embodiment is the same as that described with reference to FIG. 2.

In the present embodiment, the powder diffuser 15 may include an impact device. According to the present embodiment, the impact device included in the powder diffuser 15 may apply physical impact to the storage tank 11 to drop the powder-type medium attached to an inner wall of the storage tank 11 to the bottom of the storage tank 11 by gravity, and thus the amount of powder-type medium transferred to the powder loading unit 12 may be increased.

An operation of the impact device according to the present embodiment is the same as that described with reference to FIG. 3.

*111Because the powder inhalation device 1 according to the disclosure controls the amount of diffusion of powder transferred from the inside of the storage tank 11 to the powder loading unit 12 through the vibration device or the impact device, thereby controlling a concentration of the powder-type medium included in the air flow. As a result, a sufficient powder-type medium may be supplied to the user in accordance with the user's inhalation ability and/or inhalation timing, and reliable supply of the powder-type medium may be ensured even when the user's inhalation ability is insufficient.

In another example, at least two powder diffusers 15 may be provided, and one powder diffuser 15 may be located adjacent to the storage tank 11 and the other powder diffuser 15 may be located below the storage tank 11. That is, a plurality of powder diffusers 15 may be provided and each powder diffuser may perform a powder diffusion operation.

According to the above arrangement, the powder diffuser 15 located adjacent to the storage tank 11 may increase the amount of powder-type medium transferred from the storage tank 11 to the powder loading unit 12, and the powder diffuser 15 located below the storage tank 11 may increase the amount of powder-type medium diffused from the powder loading unit 12 toward the air flow passage 13. Therefore, the amount of powder-type medium supplied to the user may be increased.

FIG. 6 is a block diagram illustrating the powder inhalation device 1, according to an embodiment.

Referring to FIG. 6, the controller 20 may be electrically connected to the air flow sensor 14, the powder diffuser 15, an output unit 16, and the battery 30.

The air flow sensor 14 may include at least one of, but not limited to, a pressure sensor 141, a flow rate sensor 142, and a flow velocity sensor 143. A shape and structure of each sensor may be intuitively inferred from its name, and thus, a detailed description thereof will be omitted.

The pressure sensor 141 may detect a pressure of an air flow in an air flow passage.

The flow rate sensor 142 may detect a flow rate of the air flow in the air flow passage.

The flow velocity sensor 143 may detect a flow velocity of the air flow in the air flow passage.

The air flow sensor 14 may detect a user's inhalation based on at least one of a pressure change, a flow rate change, and a flow velocity change.

The air flow sensor 14 detects a physical quantity of at least one of the pressure, the flow rate, and the flow velocity of the air flow in the air flow passage, and may transmit a pressure signal, a flow rate signal, and/or a flow velocity signal to the controller 20 according to detected information.

The controller 20 may control an overall operation of the powder inhalation device 1.

The controller 20 may receive a signal from the air flow sensor 14, and may operate the powder diffuser 15 based on a change of the air flow in the air flow passage 13.

The controller 20 may operate the powder diffuser 15, based on a detection value of the physical quantity (pressure, flow rate, and/or flow velocity) detected by the signal of the air flow sensor 14.

In an embodiment, the controller 20 may include at least one processor (not shown). The processor may include an array of logic gates, or may include a combination of a general-purpose microprocessor and a memory in which a program executable in the microprocessor is stored. Also, it will be understood by one of ordinary skill in the art related to the present embodiment that the processor includes other types of hardware.

The controller 20 may control an operation of the powder diffuser 15 by controlling supply of power from the battery 30 to the powder diffuser 15. For example, the controller 20 may control power supply by controlling switching of a switching device between the battery 30 and the powder diffuser 15.

The controller 20 may analyze the detection value of the physical quantity detected by the air flow sensor 14, and may control subsequent processes.

The controller 20 may control power supplied to the powder diffuser 15 to start or end an operation of the powder diffuser 15, based on the detection value of the physical quantity detected by the air flow sensor 14.

The controller 20 controls the amount of power supplied to the powder diffuser 15 and a time during which power is supplied to the powder diffuser 15 to operate the powder diffuser 15 and adjust the amount of powder-type medium supplied to a user, based on the detection value of the physical quantity detected by the air flow sensor 14.

An embodiment may be implemented in the form of a computer-readable recording medium that includes computer-executable instructions such as program modules executed by a computer. A computer-readable medium may be an arbitrary available medium accessible by a computer, and includes all volatile and non-volatile media and separable and non-separable media. Also, examples of the computer-readable medium may include a computer storage medium and a communication medium. Examples of the computer storage medium include all volatile and non-volatile media and separable and non-separable media, which have been implemented by an arbitrary method or technology, for storing information such as computer-readable instructions, data structures, program modules, and other data. The communication medium generally includes a computer-readable instructions, a data structure, a program module, other data of a modulated data signal, or another transmission mechanism, and an example thereof includes an arbitrary information transmission medium.

The output unit 16 may provide information about a state of the powder inhalation device 1 to the user. The output unit 16 may include at least one of, but not limited to, a display unit, a haptic unit, and a sound output unit. When the display unit is implemented as a touchscreen in which a display device and a touch pad form a layer structure, the display unit may be used as an input device as well as an output device.

The display unit may visually provide information about the powder inhalation device 1 to the user. For example, the information about the powder inhalation device 1 may refer to various information such as a charge/discharge state of the battery 30 of the powder inhalation device 1, an operation state of the powder diffuser 15, or a state in which the use of the powder inhalation device 1 is limited (e.g., detection of a foreign material in the air flow passage), and the display unit may output the information to the outside. Examples of the display unit may include a liquid crystal display panel (LCD) and an organic light-emitting display panel (OLED). Also, the display unit may be an LED light-emitting device.

The haptic unit may convert an electrical signal into a mechanical stimulus or an electrical stimulus, and may tactilely provide the information about the powder inhalation device 1 to the user. For example, the haptic unit may include a motor, a piezoelectric element, or an electrical stimulation device.

The sound output unit may audibly provide the information about the powder inhalation device 1 to the user. For example, the sound output unit may convert an electrical signal into a sound signal and may output the sound signal to the outside.

The powder inhalation device 1 may further include a power conversion circuit (e.g., a direct current (DC)/DC converter) for converting power of the battery 30 and supplying it to the air flow sensor 14, the powder diffuser 15, the output unit 16, and the controller 20. Also, the powder inhalation device 1 may further include a DC/alternating current (AC) converter for converting DC power of the battery 30 into AC power.

The input unit may receive information input from the user, and may transmit the received information to the controller 20. Examples of the input unit may include, but are not limited to, a key pad, a dome switch, a touch pad (e.g., contact capacitance type, pressure resistive type, infrared (IR) detection type, surface ultrasonic wave conduction type, integral tension measuring type, or piezoelectric effect type), a jog wheel, and a jog switch.

Also, the powder inhalation device 1 may further include a connection interface such as a universal serial bus (USB) interface, and may be connected to another external device through the connection interface such as the USB interface to transmit and receive information or charge the battery 30.

The input unit may further include a photo-plethysmogram (PPG) sensor. The PPG sensor may be located outside the powder inhalation device 1.

When the user grips the powder inhalation device 1 to use the powder inhalation device 1, the PPG sensor may transmit information about the user's grip to the controller 20.

The controller 20 may receive the information about the user's grip from the PPG sensor, and may prepare to operate the powder inhalation device **1.**

The controller 20 may include a memory (not shown) or may be communicatively connected to the memory. The memory that is hardware for storing various data processed in the powder inhalation device 1 may store data processed by the controller 20 and data to be processed. The memory may include at least one type of storage medium of a flash memory type storage unit, a hard disk type storage unit, a multimedia card micro type storage unit, a card type memory (e.g., an SD or XD memory), a random-access memory (RAM), a static random-access memory (SRAM), a read-only memory (ROM), an electrically erasable programmable read-only memory (EEPROM), a programmable read-only memory (PROM), a magnetic memory, a magnetic disk, and an optical disk.

The memory may store a value of a physical quantity related to a pressure, a flow rate, and/or a flow velocity of an air flow passage and an inhalation maintenance time of the powder inhalation device 1.

The powder inhalation device may include a communication unit (not shown). The communication unit may include at least one element for communication with another electronic device. For example, the communication unit may include a short-range communication unit and a wireless communication unit.

Examples of the short-range communication unit may include, but are not limited to, a Bluetooth communication unit, a Bluetooth low energy (BLE) communication unit, a near-field communication (NFC) unit, a wireless local area network (WLAN) (Wi-Fi) communication unit, a Zigbee communication unit, an infrared data association (IrDA) communication unit, a Wi-Fi direct (WFD) communication unit, an ultra-wideband (UWB) communication unit, and an Ant+ communication unit.

Examples of the wireless communication unit may include, but are not limited to, a cellular network communication unit, an Internet communication unit, and a computer network communication unit (e.g., a LAN or a WAN). The wireless communication unit may identify and authenticate the powder inhalation device 1 in a communication network by using subscriber information (e.g., international mobile subscriber identity (IMSI)).

The battery 30 may supply power used to operate the powder inhalation device 1. The battery 30 may supply power necessary to operate elements such as the air flow sensor 14, the powder diffuser 15, the output unit 16, and the controller 20 provided in the powder inhalation device 1.

The air flow sensor 14, the powder diffuser 15, the output unit 16, and the controller 20 may receive power from the battery 30 to perform functions.

The battery 30 may be a rechargeable battery or a disposable battery. For example, the battery 30 may be, but is not limited to, a lithium polymer (LiPoly) battery.

The powder inhalation device 1 according to the disclosure may further include a power conversion circuit for converting power of the battery 30 and supplying it to each element, for example, a low-dropout (LDO) circuit or a voltage regulator circuit.

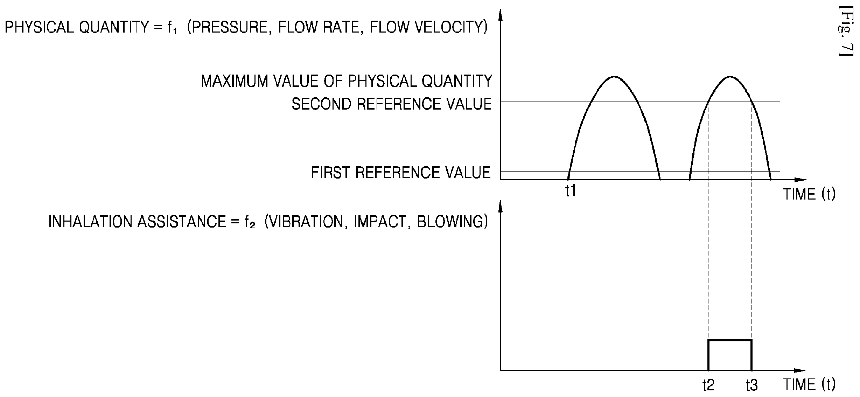

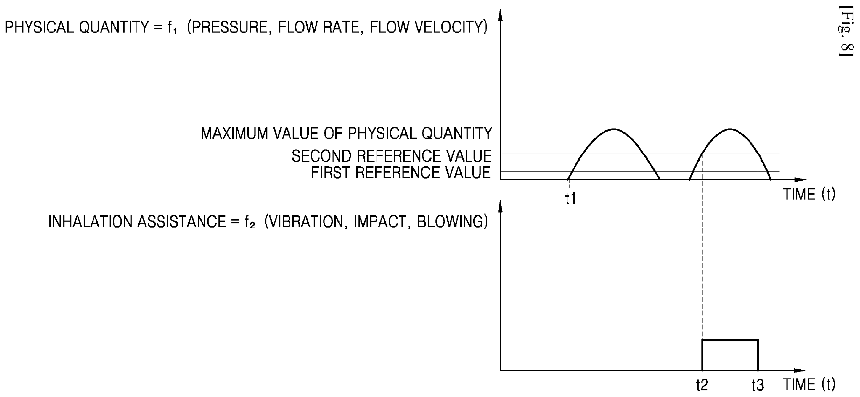

FIGS. 7 and 8 are graphs for describing a method by which the powder inhalation device 1 detects a user's inhalation intention, detects a physical quantity related to inhalation ability, and performs an inhalation assistance operation according to the detected physical quantity, according to an embodiment of the disclosure.

The graphs of FIGS. 7 and 8 will be described with reference to elements of the powder inhalation device 1 of FIGS. 1 to 6.

Referring to FIGS. 7 and 8, the powder inhalation device 1 may perform an inhalation assistance operation for a short time and/or with low intensity when a user's inhalation ability is strong, and may perform an inhalation assistance operation for a longer duration and/or with greater intensity when the user's inhalation ability is weak, which will be described below in detail.

In FIGS. 7 and 8, two graphs, that is, upper and lower graphs, are illustrated. In the two graphs, the horizontal axis represents a time.

The vertical axis of the upper graph in FIGS. 7 and 8 represents a magnitude of a physical quantity indicating the user's inhalation ability. The physical quantity indicating the user's inhalation ability may be a function (f₁=(pressure, flow rate, flow velocity)) having a pressure, a flow rate, and a flow velocity as variables.

The vertical axis of the lower graph in FIGS. 7 and 8 represents an intensity of an inhalation assistance operation. The intensity of the inhalation assistance operation may be a function (f₂=(vibration, impact, blowing)) having intensities of vibration, impact, and blowing as variables.

The controller 0 may determine the user's inhalation ability, and may control an operation of the powder diffuser 15 based on the user's inhalation ability. Because the operation of the powder diffuser 15 assists the user to inhale powder, the operation of the powder diffuser 15 may be referred to as an 'inhalation assistance function' or an 'inhalation assistance operation'.

The inhalation assistance operation of the powder diffuser 15 may be performed through vibration, impact, and/or blowing as described above. That is, the inhalation assistance operation may be performed including at least one of vibration, impact, and blowing.

When the user's inhalation is detected, the controller 20 may determine the user's inhalation ability, and may control the operation of the powder diffuser 15 according to the user's inhalation ability.

The controller 20 may compare a detection value of a physical quantity indicating the user's inhalation ability with a pre-determined value, and may control a strength of the operation and/or a duration of the operation of the powder diffuser 15 to correspond to a level of the user's inhalation ability based on a comparison result.

When the user uses the powder inhalation device 1, a pressure, a flow rate, and/or a flow velocity of air in the air flow passage 13 changes. The user's inhalation amount may be a function having a pressure, a flow rate, and/or a flow velocity as variables. For example, the inhalation amount of the user who uses the powder inhalation device 1 is proportional to the pressure, the flow rate, and the flow velocity in the air flow passage 13.

The air flow sensor 14 detects the pressure, the flow rate, and/or the flow velocity in the air flow passage 13. When a detection value of a physical quantity of the pressure, the flow rate, and/or the flow velocity detected by a signal of the air flow sensor 14 is equal to or greater than a preset first reference value, the controller 20 may determine that the user's inhalation starts.

When it is determined that the user's inhalation starts, the controller 20 may obtain a maximum value of the physical quantity detected by the signal of the air flow sensor 14.

In order to control the powder diffuser 15 according to the user's inhalation ability, the controller 20 may determine a second reference value for operating the powder diffuser 15 based on the maximum value of the physical quantity of the air flow in the air flow passage 13.

The second reference value may be a value of a physical quantity based on which the controller 20 operates the powder diffuser 15.

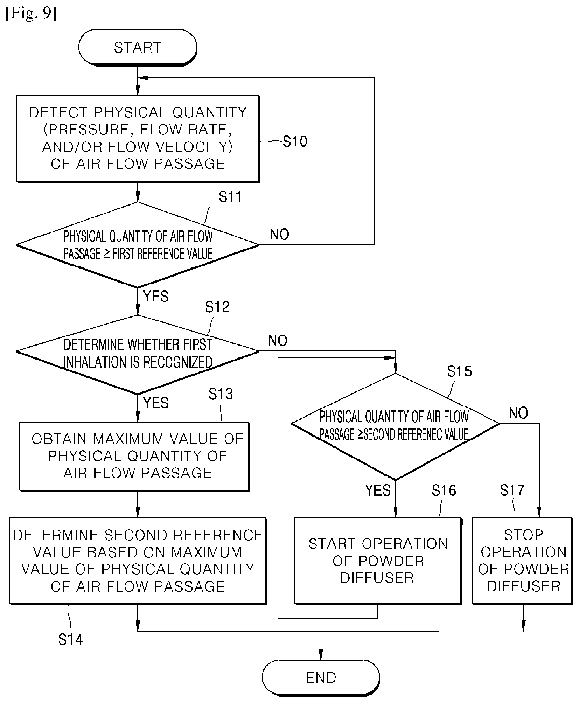

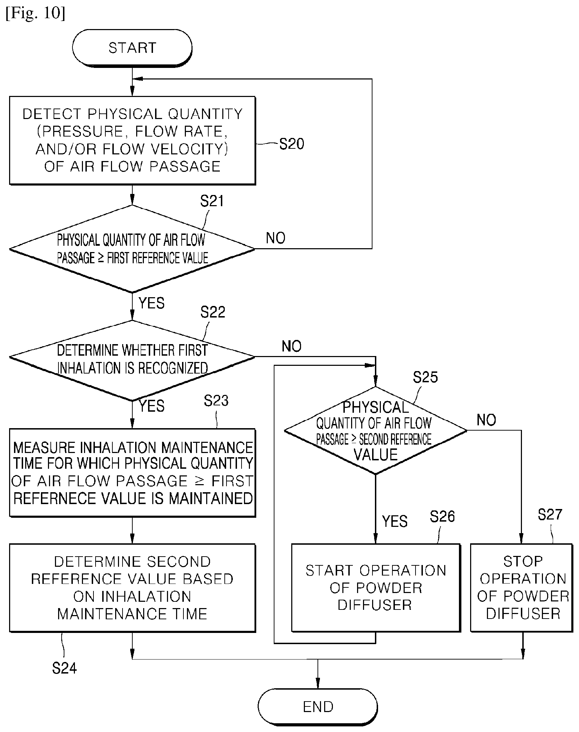

FIGS. 9 and 10 are flowcharts for describing a method of controlling a powder inhalation device, according to various embodiments of the disclosure.

A method of controlling the powder inhalation device 1 according to a user's inhalation ability will be described in detail with reference to FIGS. 9 and 10.

The method of controlling the powder inhalation device 1 of FIGS. 9 and 10 will be described with reference to the elements of the powder inhalation device 1 of FIGS. 1 to 6 and the graphs of FIGS. 7 and 8.

FIG. 9 is a flowchart for describing a process in which the controller 20 of the powder inhalation device 1 measures a maximum value of a physical quantity of the air flow passage 13 based on a change of a physical quantity (a pressure, a flow rate, and/or a flow velocity) of the air flow passage 13 detected from the air flow sensor 14, determines a second reference value based on the maximum value of the physical quantity of the air flow passage 13, and operates the powder diffuser 15 based on the second reference value, according to an embodiment.

Referring to FIG. 9, in operation S10, the air flow sensor 14 of the powder inhalation device 1 detects a physical quantity of a pressure, a flow rate, and/or a flow velocity of an air flow in the air flow passage 13.

In operation S11, the controller 20 may determine whether a detection value of the physical quantity detected by a signal of the air flow sensor 14 is equal to or greater than a preset first reference value.

A dimension or unit of the first reference value may be set according to a type of a physical quantity (a pressure, a flow rate, or a flow velocity) to be detected by the air flow sensor 14.

The first reference value may be used as a value for determining whether a user starts inhalation by using the powder inhalation device 1. The first reference value may be set as a value for detecting the user's inhalation intention. That is, when the detection value of the physical quantity of the air flow passage 13 is equal to or greater than the first reference value, it may be determined that the user intends to inhale powder by using the powder inhalation device 1.

For example, referring to the graphs of FIGS. 7 and 8, when the time reaches t1, the detection value of the physical quantity of the air flow passage 13 detected by the air flow sensor 14 is equal to or greater than the first reference value, and it may be determined that the user intends to start inhaling powder by using the powder inhalation device 1 from the time t1.

When it is determined in operation S11 that the detection value of the physical quantity of the air flow passage 13 is less than the first reference value, the controller 20 may compare the detection value of the physical quantity of the air flow passage 13 by the signal of the air flow sensor 14 with the first reference value again (return to operation S10).

When it is determined in operation S11 that the detection value of the physical quantity of the air flow passage 13 is equal to or greater than the first reference value, the controller 20 may determine that the user starts an 'inhalation operation', and may determine whether the user's 'inhalation operation' is the 'first inhalation' (operation S12).

An 'inhalation operation' may refer to an operation of the user during a period from when the physical quantity of the air flow passage becomes is equal to or greater than the first reference value to when the physical quantity decreases to the first reference value after reaching a maximum value.

An 'inhalation operation' may refer to an operation of the user during a period in which the user's inhalation intention is maintained.

The 'first inhalation' refers to a first operation of performing inhalation when the user inhales powder by using a powder inhalation device.

The 'first inhalation' may refer to inhalation during a period from when the detection value of the physical quantity of the air flow passage 13 becomes equal to or greater than the first reference value for the first time to when the detection value becomes less than the first reference value, in a case where the powder inhalation device 1 has not been used for a certain period of time.

In another example, the 'first inhalation' may refer to inhalation during a period from when the detection value of the physical quantity of the air flow passage 13 becomes equal to or greater than the first reference value for the first time to when the detection value becomes less than the first reference value, after power of the powder inhalation device 1 is turned on.

In another example, the 'first inhalation' may refer to inhalation during a period from when the detection value of the physical quantity of the air flow passage 13 becomes equal to or greater than the first reference value to when the detection value becomes less than the first reference value, after the user grips the powder inhalation device 1 and information about the user's grip is transmitted from a PPG sensor to the controller 20.

In operation S13, when it is determined that the user's inhalation operation is the first inhalation, the controller 20 may obtain a maximum value of the physical quantity of the air flow passage 13.

In operation S14, the controller 20 may determine a second reference value based on the maximum value of the physical quantity of the air flow passage 13 obtained in operation S13.

For example, the controller 20 may determine the second reference value to be proportional to the maximum value of the physical quantity of the pressure, the flow rate, and/or the flow velocity of the air flow passage 13 detected by the signal of the air flow sensor 14.

Because the maximum value of the physical quantity of the pressure, the flow rate, and/or the flow velocity of the air flow passage 13 is proportional to the user's inhalation ability, the second reference value may be determined to be proportional to the user's inhalation ability.

When it is determined in operation S12 that the user's inhalation operation is not the first inhalation, in operation S15, the controller 20 may determine whether the detection value of the physical quantity of the air flow passage 13 detected by the signal of the air flow sensor 14 is equal to or greater than the second reference value determined in operation S14.

In operation S16, the controller 20 may operate the powder diffuser 15 when the detection value of the physical quantity of the air flow passage 13 is equal to or greater than the second reference value.

Then, the controller 20 may again determine whether the detection value of the physical quantity of the air flow passage 13 detected by the signal of the air flow sensor 14 is equal to or greater than the second reference value determined in operation S14 (i.e., return to operation S15).

In operation S17, the controller 20 may stop the operation of the powder diffuser 15 when the detection value of the physical quantity of the air flow passage 13 is less than the second reference value.

The user may repeatedly perform an inhalation operation by using the powder inhalation device 1. In this regard, the controller 20 performs an operation of determining the second reference value based on the maximum value of the physical quantity of the air flow passage 13 only when the user's inhalation operation is the first inhalation (operation S15). If the user's inhalation operation is not the first inhalation (i.e., if the user's inhalation operation is the second or subsequent inhalations), the controller 20 may determine whether to operate the powder diffuser 15 based on the second reference value determined in operation S14.

The user may repeatedly perform an inhalation operation after the first inhalation, and the controller 20 may operate the powder diffuser 15 only while the detection value of the physical quantity of the air flow passage 13 is maintained greater than the second reference value in the repeated inhalation operations after the user's first inhalation, thereby performing an inhalation assistance operation of diffusing a powder-type medium transferred to the powder loading unit 12 toward the air flow passage 13.

Referring to the graphs of FIGS. 7 and 8, the controller 20 may detect the user's inhalation operation at t1, and may determine the second reference value by measuring the user's inhalation ability during the first inhalation.

When the 'first inhalation' is performed, the powder inhalation device does not operate and the user may inhale powder by using only his/her own inhalation ability. Alternatively, in another embodiment, when the 'first inhalation' is performed, the powder inhalation device may operate at a minimum strength (intensity) to assist the user in inhaling the powder.

After the 'first inhalation' ends, the 'second inhalation' in which the user performs an inhalation operation again may follow. While the 'second inhalation' is performed, the controller 20 may perform an inhalation assistance operation from t2 to t3 by operating the powder diffuser 15.

Although the graphs of FIGS. 7 and 8 show that an inhalation assistance operation is performed once (t2 to t3) in the 'second inhalation', an inhalation assistance operation based on the second reference value may be repeatedly performed according to the user's inhalation operation.

The second reference value may be determined to be proportional to a pressure, a flow rate, and/or a flow velocity in the air flow passage 13 of the user. As such, when the user's inhalation pressure, flow rate, and/or flow velocity is relatively weak, the powder diffuser 15 may operate at a lower inhalation pressure, flow rate, and/or flow velocity than that when the user's inhalation pressure, flow rate, and/or flow velocity is strong.

Referring to FIGS. 7 and 8, the maximum value of the physical quantity of FIG. 7 is greater than a maximum value of a physical quantity of the user of FIG. 8. The maximum value of the physical quantity may be proportional to the user's inhalation ability.

For example, when the user has strong inhalation ability, the second reference value may be set to a high value, and when the user has weak inhalation ability, the second reference value may be set to a low value. Accordingly, when the user has weak inhalation ability, an operation of the powder diffuser 15 starts based on the low second reference value, and thus the powder diffuser 15 may assist a powder inhalation operation of the user having weak inhalation ability for a longer time.

Referring to FIGS. 7 and 8, an inhalation assistance operation (i.e., an operation of the powder diffuser 15) may be performed from t2 to t3. A time interval between t2 to t3 may be longer when the maximum value of the physical quantity is relatively small as in FIG. 8 than when the maximum value of the physical quantity is relatively large as in FIG. 7. That is, an inhalation assistance operation may be performed longer when the maximum value of the physical quantity is smaller.

Because the maximum value of the physical quantity is proportional to the user's inhalation ability, the inhalation assistance operation time (t2 to t3) is longer when the user's inhalation ability is weak as in FIG. 8 than when the user's inhalation ability is strong as in FIG. 7.

That is, the powder diffuser 15 may operate passively when the user's inhalation ability is strong, and may operate actively when the user's inhalation ability is weak.

When the powder diffuser 15 'operates passively', it may mean that a strength of vibration generated by the powder diffuser 15 is set to be weak or an operation start time of the powder diffuser 15 is set to be late.

When the powder diffuser 'operates actively', it may mean that a strength of vibration generated by the powder diffuser 15 is set to be strong or an operation start time of the powder diffuser 15 is set to be early.

As such, a strong inhalation assistance operation is performed for a user having weak inhalation ability and a weak inhalation assistance operation is performed for a user having strong inhalation ability. As a result, a sufficient amount of powder may be supplied to the user of the powder inhalation device 1 according to the inhalation ability of the user of the powder inhalation device 1.

Because the powder diffuser 15 operates when the user performs an inhalation operation by using the powder inhalation device 1, the powder diffuser 15 may supply powder to the user according to the user's inhalation timing.

When the detection value of a physical quantity of the air flow passage 13 is equal to or greater than the first reference value, it may be determined that the user starts an inhalation operation.

Because the powder diffuser 15 does not operate directly even when the detection value of the physical quantity of the air flow passage 13 is equal to or greater than the first reference value, and operates only when the detection value is equal to or greater than the second reference value, reliable powder supply may be performed according to the user's inhalation ability.

When the user's inhalation ability is above a certain level, for example, when the user's inhalation ability exceeds an average adult level and does not require an inhalation assistance operation, there may be no need to perform the inhalation assistance operation. Accordingly, when the detection value of the physical quantity of the air flow passage 13 detected by the air flow sensor 14 is a certain value or more, the controller 20 may not operate the powder diffuser 15.

FIG. 10 is a flowchart for describing a process in which the controller 20 of the powder inhalation device 1 measures an inhalation maintenance time based on a change in a physical quantity (a pressure, a flow rate, and/or a flow velocity) of the air flow passage 13 detected from the air flow sensor 14, determines a second reference value based on the inhalation maintenance time, and operates the powder diffuser 15 based on the second reference value, according to an embodiment.

The same description as that made for the method of controlling the powder inhalation device 1 described with reference to FIG. 9 will be omitted.

Referring to FIG. 10, in operation S20, the air flow sensor 14 of the powder inhalation device 1 detects a physical quantity of a pressure, a flow rate, and/or a flow velocity of an air flow in the air flow passage 13.

In operation S21, the controller 20 may determine whether a detection value of the physical quantity detected by a signal of the air flow sensor 14 is equal to or greater than a preset first reference value.

When it is determined in operation S21 that the detection value of the physical quantity of the air flow passage 13 is less than the first reference value, the controller 20 may compare the detection value of the physical quantity of the air flow passage 13 detected by the signal of the air flow sensor 14 with the first reference value again (return to operation S20).

When it is determined in operation S21 that the detection value of the physical quantity of the air flow passage 13 is equal to or greater than the first reference value, the controller 20 may determine that a user starts an 'inhalation operation', and may determine whether the user's 'inhalation operation' is the 'first inhalation' (operation S22).

When it is determined in operation S22 that the user's 'inhalation operation' is the 'first inhalation', the controller 20 of the powder inhalation device 1 may measure a time for which the detection value of the physical quantity of the air flow passage 13 detected by the air flow sensor 14 is maintained at or above the first reference value (operation S23).

In this case, the time for which the detection value of the physical quantity of the air flow passage 13 is maintained at or above the first reference value in the 'first inhalation' may be referred to as an 'inhalation maintenance time'.

That is, a period from when the detection value of the physical quantity of the air flow passage 13 reaches the first reference value for the first time in the 'first halation' to when the detection value decreases to the first reference value after reaching a maximum value may be referred to as an inhalation maintenance time.

Because an inhalation maintenance time may be maintained longer as the user's inhalation ability is stronger, the inhalation maintenance time may be proportional to the inhalation ability.

As described above, the first reference value is a value for detecting the user's inhalation intention. In this regard, the inhalation maintenance time may be a time for which the user's intention to inhale powder by using the powder inhalation device 1 is maintained.

In another aspect, the inhalation maintenance time may be a time for which an inhalation operation is maintained.

In operation S24, the controller 20 of the powder inhalation device 1 according to an embodiment may determine a second reference value based on the inhalation maintenance time measured in the air flow passage 13 in operation S23.

For example, the controller 20 may determine the second reference value to be proportional to the inhalation maintenance time.

Because the inhalation maintenance time of the air flow passage 13 is proportional to the user's inhalation ability, the second reference value may be determined to be proportional to the user's inhalation ability.

For example, when the user has strong inhalation ability, the second reference value may be set to a high value, and when the user has weak inhalation ability, the second reference value is set to a low value. Accordingly, when the user has weak inhalation ability, because an operation of the powder diffuser 15 starts based on the second reference value set to a low value, the powder diffuser 15 may assist a powder inhalation operation of the user having weak inhalation ability for a longer time.

When it is determined in operation S22 that the user's inhalation operation is not the 'first inhalation', in operation S25, the controller 20 may determine whether the detection value of the physical quantity of the air flow passage 13 detected by the signal of the air flow sensor 14 is equal to or greater than the second reference value determined in operation S24.

In operation S26, the controller 20 may operate the powder diffuser 15 when the detection value of the physical quantity of the air flow passage 13 is equal to or greater than the second reference value.

Then, the controller 20 may again determine whether the detection value of the physical quantity of the air flow passage 13 detected by the signal of the air flow sensor 14 is equal to or greater than the second reference value determined in operation S24 (i.e., return to operation S25).

In operation S27, the controller 20 may stop the operation of the powder diffuser 15 when the detection value of the physical quantity of the air flow passage 13 is less than the second reference value.

The user repeatedly performs an inhalation operation by using the powder inhalation device 1. In this regard, the controller 20 performs an operation of determining the second reference value based on the maximum value of the physical quantity of the air flow passage 13 only when the user's inhalation operation is the 'first inhalation' (operation S25). If the user's inhalation operation is not the first inhalation (i.e., if the user's inhalation operation is the second or subsequent inhalations), the controller 20 may determine whether to operate the powder diffuser 15 based on the second reference value determined in operation S24.

The user may repeatedly perform an inhalation operation after the first inhalation, and the controller 20 may operate the powder diffuser 15 only while the detection value of the physical quantity of the air flow passage 13 during one inhalation operation after the user's first inhalation is maintained greater than the second reference value, thereby performing a powder inhalation assistance operation of diffusing a powder-type medium transferred to the powder loading unit 12 toward the air flow passage 13.

As such, a strong inhalation assistance operation is performed for a user having weak inhalation ability and a weak inhalation assistance operation is performed for a user having strong inhalation ability. As a result, a sufficient amount of powder may be supplied to the user of the powder inhalation device 1 according to the inhalation ability of the user of the powder inhalation device 1.

Because the powder diffuser 15 operates as described above, the powder inhalation device 1 according to the disclosure may supply sufficient powder to the user even when the user's inhalation ability is insufficient. Also, the powder inhalation device 1 according to the disclosure may supply sufficient powder to the user in accordance with the user's inhalation ability.

The method of controlling the powder diffuser 15 according to a pressure, a flow rate, or a flow velocity of the air flow passage 13, or an inhalation maintenance time described with reference to FIGS. 9 and 10 may be performed in dependently or in combination.

Dimensions and units of a measurement target (pressure, flow rate, flow velocity, or inhalation time) and a criterion of an intensity of an inhalation assistance operation (pressure, flow rate, or flow velocity) may be different from each other.

For example, the controller may measure the user's inhalation ability based on the maximum value of the detection value of the pressure of the air flow passage 13 detected by the air flow sensor 14, and then may determine the second reference value that is a value for determining an operation of the powder diffuser 15 based on the flow velocity of the air flow passage 13.

In another example, the controller 20 may measure the user's inhalation ability in proportion to the inhalation maintenance time, and then may determine the second reference value of the air flow passage 13 based on the pressure of the air flow passage 13.

There may be one or more measurement targets, and there may be one or more criteria of an intensity of an inhalation assistance operation. That is, inhalation ability may be a function of multiple variables, and an intensity of an inhalation assistance operation may also be a function of multiple variables.

For example, the controller 20 may measure the user's inhalation ability by considering both the maximum value of the pressure and the maximum value of the flow velocity of the air flow passage 13, may determine a reference pressure and a reference flow velocity, and then may operate the powder diffuser 15 when they reach the reference pressure and the reference velocity.

In another example, the controller 20 may measure the maximum value of the pressure of the air flow passage 13 and the inhalation maintenance time, and then may determine a reference pressure based on the maximum value of the pressure and the inhalation maintenance time.

The setting of a measurement criterion of inhalation ability and/or a criterion of an intensity of an inhalation assistance operation may be modified, combined, and selected in various ways.

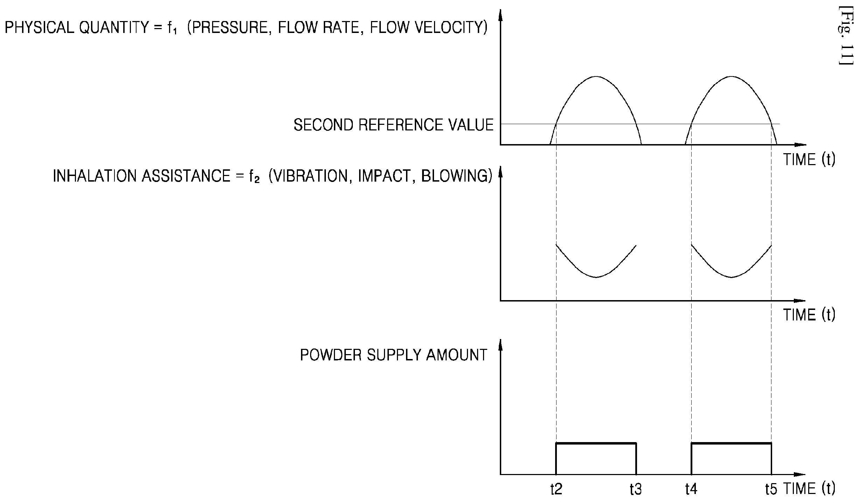

FIG. 11 is a graph for describing a method of supplying sufficient powder in accordance with a user's inhalation ability, according to an embodiment.

FIG.11 is a graph for describing a specific embodiment in which determining a second reference value (a pressure, a flow rate, and/or a flow velocity) is determined according to an embodiment described with reference to FIGS. 9 and 10 and then an inhalation assistance operation is performed based on the second reference value.

In FIG. 11, three graphs, that is, upper, middle, and lower graphs are illustrated. In the three graphs, the horizontal axis represents a time.

The vertical axis of the upper graph of FIG. 11 represents a magnitude of a physical quantity indicating the user's inhalation ability. The physical quantity indicating the user's inhalation ability may be a function (f₁=(pressure, flow rate, flow velocity)) having a pressure, a flow rate, and a flow velocity as variables.

The vertical axis of the middle graph of FIG. 1 represents an intensity of an inhalation assistance operation. The intensity of the inhalation assistance operation may be a function (f₂=(vibration, impact, blowing)) having intensities of vibration, impact, and blowing as variables.

The vertical axis of the lower graph of FIG. 11 represents a powder supply amount. The powder supply amount refers to the amount of powder actually supplied to the user by the inhalation assistance operation.

As described above, the inhalation assistance operation through the powder diffuser 15 may be performed by any one of the vibration device 15a, the impact device 15b, and the blower 15c, or a combination thereof.

The amount of powder diffusion by the powder diffuser 15 may be controlled by adjusting a frequency or an amplitude of vibration, by adjusting a frequency and strength of impact, or by adjusting a strength of blowing or an open area of an opening.

Referring to FIG. 11, the amount of inhalation (pressure, flow rate, and/or flow velocity) of the user using the powder inhalation device 1 increases at the beginning and then decreases after a maximum value.

If the controller 0 continuously operates the powder diffuser 15 at the same strength, the powder inhalation assistance operation may be maintained at the same intensity.

In this case, the amount of powder supplied to the user may not be constant because the amount of inhalation of the user changes over time.

In this regard, if an operation of the powder diffuser 15 is controlled by considering the increase and decrease in the user's inhalation amount, a sufficient amount of powder may be more effectively provided to the user.

Referring to FIG. 11, the controller 20 may control the powder diffuser 15 by considering the increase and decrease in the user's inhalation amount over time.

In detail, the controller 20 may control an intensity of an operation of the powder diffuser 15 to gradually decrease during a time from when a detection value of the physical quantity of the air flow passage 13 detected by the air flow sensor 14 is equal to or greater than a second reference value to when the detection value reaches a maximum value. Also, the controller 20 may control an intensity of an operation of the powder diffuser 15 to gradually increase during a time from when the detection value of the physical quantity of the air flow passage 13 reaches the maximum value to when the detection value is reduced to the second reference value again.

In another aspect, the controller 20 may control the intensity of the operation of the powder diffuser 15 to be inversely proportional to the detection value of the physical quantity of the air flow passage 13 detected by the air flow sensor 14.

As such, even though the user's inhalation amount increases and decreases over time, a strong inhalation assistance operation is performed when the user's inhalation amount is weak and a weak inhalation assistance operation is performed when the user's inhalation amount is strong. As a result, the amount of powder supplied to the user may be maintained constant for one inhalation operation (see the lower graph of FIG. 11).

Accordingly, the user of the powder inhalation device 1 may receive a sufficient amount of powder for each inhalation operation according to the user's inhalation operation timing.

The descriptions of the above-described embodiments are merely examples, and it will be understood by one of ordinary skill in the art that various changes and equivalents thereof may be made. Therefore, the scope of the disclosure should be defined by the appended claims.

## Claims

1. A powder inhalation device (1) comprising:
a storage tank (11) configured to store a medium in a powder form;
a powder loading unit (12) configured to receive the medium from the storage tank (11);
an air flow passage (13) through which an air flow comprising a mixture of air and the medium transferred to the powder loading unit (12) flows;
an air flow sensor (14) configured to detect a change in the air flow of the air flow passage (13);
a powder diffuser (15) configured to diffuse the medium transferred to the powder loading unit (12) toward the air flow passage (13); and
a controller (20) configured to receive a signal from the air flow sensor (14), and operate the powder diffuser (15) based on the change in the air flow of the air flow passage,
**characterized in that** the controller (20) is configured to control the amount of power supplied to the powder diffuser (15) and a time during which the power is supplied to the powder diffuser (15), such that the amount of the medium is adjusted based on a physical quantity of at least one of a pressure, a flow rate, and a flow velocity of the air flow of the air flow passage (13), detected by the air flow sensor (14).

2. The powder inhalation device of claim 1, wherein the controller is further configured to, when a detection value of the physical quantity detected by the air flow sensor is equal to or greater than a first reference value, obtain a maximum value of the physical quantity of the air flow of the air flow passage based on the signal of the air flow sensor.

3. The powder inhalation device of claim 2, wherein the controller is further configured to:
determine a second reference value for operating the powder diffuser, based on the maximum value of the physical quantity of the air flow of the air flow passage, and
operate the powder diffuser when the detection value of the air flow detected by the signal of the air flow sensor is equal to or greater than the second reference value, and
stop an operation of the powder diffuser when the detection value of the air flow detected by the signal of the air flow sensor is less than the second reference value.

4. The powder inhalation device of claim 1, wherein the controller is further configured to measure an inhalation maintenance time for which a detection value of the physical quantity detected by the signal of the air flow sensor is maintained at or above a first reference value.

5. The powder inhalation device of claim 4, wherein the controller is further configured to:
determine a second reference value for operating the powder diffuser, based on the inhalation maintenance time, and
operate the powder diffuser when the detection value of the air flow detected by the signal of the air flow sensor is equal to or greater than the second reference value, and
stop an operation of the powder diffuser when the detection value is less than the second reference value.

6. The powder inhalation device of claim 1, wherein the powder loading unit comprises a vibration plate configured to vibrate the medium, and
the powder diffuser comprises a vibration device configured to diffuse the medium transferred to the powder loading unit toward the air flow passage by vibrating the vibration plate.

7. The powder inhalation device of claim 1, wherein the powder diffuser comprises an impact device configured to diffuse the medium transferred to the powder loading unit toward the air flow passage by applying physical impact to the powder loading unit.

8. The powder inhalation device of claim 1, wherein the powder diffuser comprises a blower configured to diffuse the medium transferred to the powder loading unit toward the air flow passage by generating a flow of air.

9. A method of controlling a powder inhalation device (1), the method comprising:
an inhalation ability measurement step of detecting a change in an air flow in an air flow passage (13) which provides fluid communication between a powder loading unit (12) configured to receive a medium in a powder form and an outside of the powder inhalation device (1); and
a powder inhalation assistance step of diffusing the medium transferred to the powder loading unit (12) toward the air flow passage (13) by operating a powder diffuser (15) based on the change in the air flow in the air flow passage (13),
**characterized in that** the inhalation ability measurement step further comprises detecting (S20), by using an air flow sensor (14), a physical quantity of at least one of a pressure, a flow rate, and a flow velocity of the air flow in the air flow passage (13),
wherein the powder inhalation assistance step further comprises controlling the amount of power supplied to the powder diffuser (15) and a time during which the power is supplied to the powder diffuser (15) such that the amount of the medium is adjusted based on the physical quantity detected by the air flow sensor (14).

10. The method of claim 9, wherein the inhalation ability measurement step further comprises obtaining a maximum value of the physical quantity of the air flow in the air flow passage when a detection value of the detected physical quantity is equal to or greater than a first reference value.

11. The method of claim 10, wherein
the inhalation ability measurement step further comprises determining a second reference value based on the maximum value of the physical quantity of the air flow in the air flow passage, and
the powder inhalation assistance step further comprises operating the powder diffuser when the detection value of the physical quantity of the air flow in the air flow passage is equal to or greater than the second reference value, and stopping an operation of the powder diffuser when the detection value of the physical quantity in the air flow passage is less than the second reference value.

12. The method of claim 9, wherein the inhalation ability measurement step further comprises measuring an inhalation maintenance time for which a detection value of the detected physical quantity is maintained at or above a first reference value.

## Patentansprüche

1. Pulverinhalationsvorrichtung (1), die Folgendes umfasst:
einen Vorratsbehälter (11), der dazu konfiguriert ist, ein Medium in Pulverform aufzubewahren;
eine Pulverladeeinheit (12), die dazu konfiguriert ist, das Medium aus dem Vorratsbehälter (11) aufzunehmen;
ein Luftströmungskanal (13), durch den eine Luftströmung strömt, die eine aus der Pulverladeeinheit (12) übertragene Mischung aus Luft und dem Medium umfasst;
einen Luftströmungssensor (14), der dazu konfiguriert ist, eine Änderung der Luftströmung in dem Luftströmungskanal (13) zu detektieren;
einen Pulverzerstäuber (15), der dazu konfiguriert ist, das von der Pulverladeeinheit (12) übertragene Medium in Richtung des Luftströmungskanals (13) zu zerstäuben; und
eine Steuerung (20), die dazu konfiguriert ist, ein Signal von dem Luftströmungssensor (14) zu empfangen und den Pulverzerstäuber (15) basierend auf der Änderung der Luftströmung des Luftströmungskanals zu betreiben,
**dadurch gekennzeichnet, dass** die Steuerung (20) dazu konfiguriert ist, die Menge des dem Pulverzerstäuber (15) zugeführten Pulvers und einen Zeitraum, während dem das Pulver dem Pulverzerstäuber (15) zugeführt wird, derart zu steuern, dass die Menge des Mediums basierend auf einer von dem Luftströmungssensor (14) detektierten physikalischen Größe eines Drucks und/oder einer Strömungsrate und/oder einer Strömungsgeschwindigkeit der Luftströmung des Luftströmungskanals (13) angepasst wird.

2. Pulverinhalationsvorrichtung nach Anspruch 1, wobei die Steuerung ferner dazu konfiguriert ist, einen maximalen Wert der physikalischen Größe der Luftströmung des Luftströmungskanals basierend auf dem Signal des Luftströmungssensors zu erhalten, wenn ein Detektionswert der von dem Luftströmungssensor detektierten physikalischen Größe größer oder gleich einem ersten Referenzwert ist.

3. Pulverinhalationsvorrichtung nach Anspruch 2, wobei die Steuerung ferner für Folgendes konfiguriert ist:
Bestimmen eines zweiten Referenzwerts zum Betreiben des Pulverzerstäubers basierend auf dem maximalen Wert der physikalischen Größe der Luftströmung des Luftströmungskanals, und
Betreiben des Pulverzerstäubers, wenn der Detektionswert der vom Signal des Luftströmungssensors detektierten Luftströmung größer gleich dem zweiten Referenzwert ist, und
Anhalten eines Betriebs des Pulverzerstäubers, wenn der Detektionswert der vom Signal des Luftströmungssensors detektierten Luftströmung kleiner als der zweite Referenzwert ist.

4. Pulverinhalationsvorrichtung nach Anspruch 1, wobei die Steuerung ferner dazu konfiguriert ist, eine Inhalations-Haltezeit zu messen, bei der ein Detektionswert der vom Signal des Luftströmungssensors detektierten physikalischen Größe auf oder über einem ersten Referenzwert gehalten wird.

5. Pulverinhalationsvorrichtung nach Anspruch 4, wobei die Steuerung ferner für Folgendes konfiguriert ist:
Bestimmen eines zweiten Referenzwerts zum Betreiben des Pulverzerstäubers basierend auf der Inhalations-Haltezeit, und
Betreiben des Pulverzerstäubers, wenn der Detektionswert der vom Signal des Luftströmungssensors detektierten Luftströmung größer gleich dem zweiten Referenzwert ist, und
Anhalten eines Betriebs des Pulverzerstäubers, wenn der Detektionswert kleiner als der zweite Referenzwert ist.

6. Pulverinhalationsvorrichtung nach Anspruch 1, wobei die Pulverladeeinheit eine Vibrationsplatte umfasst, die dazu konfiguriert ist, das Medium zum Schwingen zu bringen, und
der Pulverzerstäuber eine Vibrationsvorrichtung umfasst, die dazu konfiguriert ist, das an die Pulverladeeinheit übertragene Medium in Richtung des Luftströmungskanals zu zerstäuben, indem sie die Vibrationsplatte zum Schwingen bringt.

7. Pulverinhalationsvorrichtung nach Anspruch 1, wobei der Pulverzerstäuber eine Schlagvorrichtung umfasst, die dazu konfiguriert ist, das an die Pulverladeeinheit übertragene Medium in Richtung des Luftströmungskanals zu zerstäuben, indem sie einen physischen Schlag auf die Pulverladeeinheit ausübt.

8. Pulverinhalationsvorrichtung nach Anspruch 1, wobei der Pulverzerstäuber ein Gebläse umfasst, das dazu konfiguriert ist, das an die Pulverladeeinheit übertragene Medium in Richtung des Luftströmungskanals zu zerstäuben, indem es eine Luftströmung erzeugt.

9. Verfahren zum Steuern einer Pulverinhalationsvorrichtung (1), wobei das Verfahren Folgendes umfasst:
einen Inhalationsfähigkeitsmessschritt des Detektierens einer Änderung einer Luftströmung in einem Luftströmungskanal (13), der eine Fluidverbindung zwischen einer Pulverladeeinheit (12), die dazu konfiguriert ist, ein Medium in Pulverform aufzunehmen, und einer Außenseite der Pulverinhalationsvorrichtung (1) bereitstellt; und
einen Pulverinhalationshilfsschritt des Zerstäubens des zur Pulverladeeinheit (12) übertragenen Mediums in Richtung des Luftströmungskanals (13) durch das Betreiben eines Pulverzerstäubers (15) basierend auf der Änderung der Luftströmung in dem Luftströmungskanal (13),
**dadurch gekennzeichnet, dass** der Inhalationsfähigkeitsmessschritt ferner das Detektieren (S20) einer physikalischen Größe eines Drucks und/oder einer Strömungsrate und/oder einer Strömungsgeschwindigkeit der Luftströmung in dem Luftströmungskanal (13) mithilfe eines Luftströmungssensors (14) umfasst,
wobei der Pulverinhalationshilfsschritt ferner das Steuern der Menge des dem Pulverzerstäuber (15) zugeführten Pulvers und eines Zeitraums, während dem das Pulver dem Pulverzerstäuber (15) zugeführt wird, derart umfasst, dass die Menge des Mediums basierend auf der von dem Luftströmungssensor (14) detektierten physikalischen Größe angepasst wird.

10. Verfahren nach Anspruch 9, wobei der Inhalationsfähigkeitsmessschritt ferner das Erhalten eines maximalen Werts der physikalischen Größe der Luftströmung in dem Luftströmungskanal umfasst, wenn ein Detektionswert der detektierten physikalischen Größe größer gleich einem ersten Referenzwert ist.

11. Verfahren nach Anspruch 10, wobei
der Inhalationsfähigkeitsmessschritt ferner das Bestimmen eines zweiten Referenzwerts basierend auf dem maximalen Wert der physikalischen Größe der Luftströmung in dem Luftströmungskanal umfasst, und
der Pulverinhalationshilfsschritt ferner das Betreiben des Pulverzerstäubers, wenn der Detektionswert der physikalischen Größe der Luftströmung in dem Luftströmungskanal größer gleich dem zweiten Referenzwert ist, und das Anhalten eines Betriebs des Pulverzerstäubers umfasst, wenn der Detektionswert der physikalischen Größe in dem Luftströmungskanal kleiner als der zweite Referenzwert ist.

12. Verfahren nach Anspruch 9, wobei der Inhalationsfähigkeitsmessschritt ferner das Messen einer Inhalations-Haltezeit umfasst, bei der ein Detektionswert der detektierten physikalischen Größe auf oder über einem ersten Referenzwert gehalten wird.

## Revendications

1. Dispositif d'inhalation de poudre (1) comprenant :
un réservoir de stockage (11) configuré pour stocker un produit sous forme de poudre ;
une unité de chargement de poudre (12) configurée pour recevoir le produit à partir du réservoir de stockage (11) ;
un passage d'écoulement d'air (13) à travers lequel s'écoule un flux d'air comprenant un mélange d'air et de produit transféré vers l'unité de chargement de poudre (12) ;
un capteur de flux d'air (14) configuré pour détecter un changement dans le flux d'air du passage d'écoulement d'air (13) ;
un diffuseur de poudre (15) configuré pour diffuser le produit transféré vers l'unité de chargement de poudre (12) vers le passage d'écoulement d'air (13) ; et
un contrôleur (20) configuré pour recevoir un signal à partir du capteur de flux d'air (14), et pour faire fonctionner le diffuseur de poudre (15) sur la base du changement dans le flux d'air du passage d'écoulement d'air,
**caractérisé en ce que** le contrôleur (20) est configuré pour contrôler le volume de poudre fourni au diffuseur de poudre (15) et une durée pendant laquelle la poudre est fournie au diffuseur de poudre (15), de telle façon que le volume du produit est ajusté sur la base d'une quantité physique de l'un au moins parmi une pression, un débit et une vitesse d'écoulement du flux d'air du passage d'écoulement d'air (13), détecté par le capteur de flux d'air (14).

2. Dispositif d'inhalation de poudre selon la revendication 1, dans lequel le contrôleur est en outre configuré pour obtenir une valeur maximale de la quantité physique du flux d'air du passage d'écoulement d'air, sur la base du signal du capteur de flux d'air, lorsqu'une valeur de détection de la quantité physique détectée par le capteur de flux d'air est égale ou supérieure à une première valeur de référence.

3. Dispositif d'inhalation de poudre selon la revendication 2, dans lequel le contrôleur est en outre configuré pour :
déterminer une deuxième valeur de référence pour faire fonctionner le diffuseur de poudre, sur la base de la valeur maximale de la quantité physique du flux d'air du passage d'écoulement d'air, et
faire fonctionner le diffuseur de poudre lorsque la valeur de détection du flux d'air détecté par le signal du capteur de flux d'air est égale ou supérieure à la deuxième valeur de référence, et
arrêter le fonctionnement du diffuseur de poudre lorsque la valeur de détection du flux d'air détecté par le signal du capteur de flux d'air est inférieure à la deuxième valeur de référence.

4. Dispositif d'inhalation de poudre selon la revendication 1, dans lequel le contrôleur est en outre configuré pour mesurer un temps de maintien d'inhalation pendant lequel une valeur de détection de la quantité physique détectée par le signal du capteur de flux d'air est maintenue à une première valeur de référence ou au-dessus.

5. Dispositif d'inhalation de poudre selon la revendication 4, dans lequel le contrôleur est en outre configuré pour :
déterminer une deuxième valeur de référence pour le fonctionnement du diffuseur de poudre, sur la base du temps de maintien d'inhalation, et
faire fonctionner le diffuseur de poudre lorsque la valeur de détection du flux d'air détecté par le signal du capteur de flux d'air est égale ou supérieure à la deuxième valeur de référence, et
arrêter le fonctionnement du diffuseur de poudre lorsque la valeur de détection est inférieure à la deuxième valeur de référence.

6. Dispositif d'inhalation de poudre selon la revendication 1, dans lequel l'unité de chargement de poudre comprend une plaque de vibration configurée pour faire vibrer le produit, et
le diffuseur de poudre comprend un dispositif de vibration configuré pour diffuser le produit transféré vers l'unité de chargement de poudre vers le passage d'écoulement d'air en faisant vibrer la plaque de vibration.

7. Dispositif d'inhalation de poudre selon la revendication 1, dans lequel le diffuseur de poudre comprend un dispositif d'impact configuré pour diffuser le produit transféré vers l'unité de chargement de poudre vers le passage d'écoulement d'air en appliquant un impact physique à l'unité de chargement de poudre.

8. Dispositif d'inhalation de poudre selon la revendication 1, dans lequel le diffuseur de poudre comprend un souffleur configuré pour diffuser le produit transféré vers l'unité de chargement de poudre vers le passage d'écoulement d'air en générant un flux d'air.

9. Procédé de commande d'un dispositif d'inhalation de poudre (1), le procédé comprenant :
une étape de mesure de capacité d'inhalation consistant à détecter un changement dans un flux d'air dans un passage d'écoulement d'air (13) fournissant une communication fluidique entre une unité de chargement de poudre (12) configurée pour recevoir un produit sous forme de poudre et un extérieur du dispositif d'inhalation de poudre (1) ; et
une étape d'aide à l'inhalation de poudre consistant à diffuser le produit transféré vers l'unité de chargement de poudre (12) vers le passage d'écoulement d'air (13) en faisant fonctionner un diffuseur de poudre (15) sur la base du changement dans le flux d'air dans le passage d'écoulement d'air (13),
**caractérisé en ce que** l'étape de mesure de capacité d'inhalation comprend en outre la détection (S20), à l'aide d'un capteur de flux d'air (14), d'une quantité physique de l'un au moins parmi une pression, un débit et une vitesse d'écoulement du flux d'air dans le passage d'écoulement d'air (13),
dans lequel l'étape d'aide à l'inhalation de poudre comprend en outre le contrôle du volume de poudre fourni au diffuseur de poudre (15) et d'une durée pendant laquelle la poudre est fournie au diffuseur de poudre (15), de telle façon que le volume du produit est ajusté sur la base de la quantité physique détectée par le capteur de flux d'air (14).

10. Procédé selon la revendication 9, dans lequel l'étape de mesure de la capacité d'inhalation comprend en outre l'obtention d'une valeur maximale de la quantité physique du flux d'air dans le passage d'écoulement d'air lorsqu'une valeur de détection de la quantité physique détectée est égale ou supérieure à une première valeur de référence.

11. Procédé selon la revendication 10, dans lequel
l'étape de mesure de la capacité d'inhalation comprend en outre la détermination d'une deuxième valeur de référence sur la base de la valeur maximale de la quantité physique du flux d'air dans le passage d'écoulement d'air, et
l'étape d'aide à l'inhalation de poudre comprend en outre le fonctionnement du diffuseur de poudre lorsque la valeur de détection de la quantité physique du flux d'air dans le passage d'écoulement d'air est égale ou supérieure à la deuxième valeur de référence, et l'arrêt d'un fonctionnement du diffuseur de poudre lorsque la valeur de détection de la quantité physique dans le passage d'écoulement d'air est inférieure à la deuxième valeur de référence.

12. Procédé selon la revendication 9, dans lequel l'étape de mesure de la capacité d'inhalation comprend en outre la mesure d'un temps de maintien d'inhalation pendant lequel une valeur de détection de la quantité physique détectée est maintenue à une première valeur de référence ou au-dessus.
